# FASCICULE DE BREVET EUROPEEN

(11) **EP 2 585 577 B1**
(45) Date de publication et mention de la délivrance du brevet: **04.03.2015**
(21) Numéro de dépôt: 11723483.1
(22) Date de dépôt: 07.06.2011
(51) Int. Cl.: C12M 1/22

(54) **BOITE DE PETRI POURVUE DE MOYENS FORMANT TEMOINS D'UTILISATION**
PETRISCHALE MIT VORRICHTUNG FÜR NUTZUNGSNACHWEIS
PETRI DISH WITH MEANS THAT PROVIDE EVIDENCE OF USE

(30) Priorité: 22.06.2010 FR 1054957
(43) Date de publication de la demande: 01.05.2013
(73) Titulaire: AES Chemunex, 35270 Combourg (FR)
(72) Inventeur: HUET, Stéphane, F-35170 Bruz (FR); THEPAUT, Jérôme, F-35190 Quebriac (FR); GINCHELEAU, Christophe, F-35000 Rennes (FR); SIMON, Frédéric, F-35270 Combourg (FR); REVERDY, Frank, F-35270 Meillac (FR)
(74) Mandataire: Regimbeau
(86) Numéro de dépôt international: PCT/EP2011/059366
(87) Numéro de publication internationale: WO 2011/160942

(56) Documents cités:
- EP-A1- 1 035 201
- EP-A2- 0 171 174
- GB-A- 2 106 083

## Description

La présente invention concerne une boite de Petri pour la culture des microorganismes, comprenant un réceptacle de forme de révolution recevant un substrat nutritif et sur lequel repose un couvercle.

Une particularité de cette boite de Petri, dans un mode de réalisation spécifique, réside dans la présence de plusieurs saillies ou doigts en périphérie du couvercle, et de "points d'accroche" en périphérie du réceptacle recevant les saillies du couvercle.

L'homme de l'art dans le domaine des techniques d'analyses microbiologiques utilise des boites de Petri de différentes tailles pour cultiver les microorganismes (diamètres variant de 50 à 150 mm généralement). Les matières les plus courantes utilisées pour la fabrication de ces boites sont les matières plastiques transparentes telles que le polystyrène (PS) cristal.

Il existe deux types généraux de boite de Petri, à savoir les boites à ergots dont le réceptacle présente des saillies sur lesquelles repose le couvercle, permettant ainsi un échange de gaz avec l'espace intérieur de la boite, et les boites sans ergots ne permettant pas l'échange de gaz avec l'espace intérieur de la boite.

L'homme de l'art connait l'intérêt de la gestion des échanges gazeux pour la culture de microbiologie.

On citera comme exemples de fonctions remplies par ces ergots :
- l'échange gazeux nécessaire au séchage des boites lors du remplissage à chaud du substrat nutritif (gélose) dans le réceptacle de la boite de Petri,
- la ventilation permettant le remplacement de l'oxygène présent dans l'atmosphère de l'espace interne de la boite pour la culture des germes anaérobies.

Dans le cas des boites sans ergots, le couvercle repose directement sur le réceptacle inférieur, limitant ainsi les échanges gazeux. Dans ce cas, le dessèchement du substrat nutritif est plus faible et permet ainsi de conserver le substrat nutritif plus longtemps.

L'absence d'ergots permet également d'augmenter le temps d'incubation de la boite sans dessèchement, garantissant ainsi une meilleure croissance de microorganismes "stressés" ou à croissance lente.

Certains types de boite de Petri permettent par ailleurs de fermer la boite après prélèvement par un système de verrouillage (couvercle attenant par serrage mécanique ou verrouillage mécanique par rotation ou clipage) afin que la boite ne s'ouvre pas d'elle-même pour limiter les risques de contaminations ultérieures après prélèvement.

Dans le cadre de la présente invention, le terme "prélèvement" a la définition suivante :
- dans le cas des contrôles de surface, le prélèvement consiste à appliquer la gélose (substrat nutritif) contenue dans la boîte sur la surface à contrôler ;
- dans le cas des contrôles d'air, le prélèvement consiste à ouvrir la boîte pour collecter l'air à analyser sur la gélose (soit par impaction ou par sédimentation passive de l'air sur la boîte) ;
- dans le cas d'un contrôle microbiologique d'un produit ou d'une culture de microorganisme, il s'agira d'ensemencer la gélose avec la souche ou le produit à mettre en culture.

Toutefois, si les solutions proposées jusqu'ici permettent de verrouiller ladite boite en cas de chute ou de malveillance, il est impossible de garantir que la boite réceptionnée pour la mise en culture n'a pas été ouverte et par conséquent il n'est pas possible de garantir la sécurité du résultat et l'absence de "recontamination" induisant de fait des résultats faussement positifs.

Un exemple d'une telle boîte de Petri est illustré dans le document EP-0 171 174.

La présente invention vise à résoudre ces difficultés.

En d'autres termes, son objectif est de fournir une boîte de Petri qui permette d'assurer une certaine traçabilité des différentes opérations de manipulation qui ont été opérées sur le couvercle, relativement au réceptacle (ou inversement), et tout particulièrement quand ceux-ci ont été préalablement verrouillés.

Ainsi, à titre d'exemple, il existe une attente pour une boîte de Petri qui, alors que les couvercle et réceptacle occupent une position interdisant les échanges gazeux, informent immédiatement l'opérateur d'une manipulation malencontreuse au cours de laquelle ce couvercle et ce réceptacle auraient été séparés, puis ramenés dans la position initiale.

Ainsi, on a donc affaire, selon l'invention à une Boite de Petri, qui est constituée d'un réceptacle et d'un couvercle complémentaire, qui ont tous les deux une forme de révolution et qui sont chacun délimités par une paroi de fond et au moins un muret périphérique,
- un muret du réceptacle ou, respectivement, du couvercle portant au moins deux doigts équidistants angulairement, généralement parallèles à la paroi de fond, qui font saillie radialement en direction du muret du couvercle ou, respectivement du réceptacle,
- tandis que le muret dépourvu de doigts porte un même nombre d'éléments de verrouillage pourvus d'un chemin de came apte à recevoir lesdits doigts,
- ce réceptacle et ce couvercle pouvant occuper sélectivement l'une ou l'autre des positions différentes successives indiquées ci-dessous :
   a/ une "première position" dite "préalable non verrouillée", dans laquelle lesdits doigts ne sont pas reçus dans lesdits éléments de verrouillage ;
   b/ au moins une "position intermédiaire" choisie parmi :
- une position "verrouillée non ventilée", dans laquelle lesdits doigts sont reçus dans lesdits éléments de verrouillage et dans laquelle le réceptacle est en contact intime avec le couvercle, de sorte que l'espace interne qu'ils délimitent est isolé du milieu extérieur, et
- une position "verrouillée et ventilée" dans laquelle lesdits doigts sont reçus dans lesdits éléments de verrouillage et dans laquelle le réceptacle n'est pas en contact intime avec le couvercle, de sorte que l'espace interne qu'ils délimitent n'est pas isolé du milieu extérieur ;
- c/ une "position finale" dite "de lecture", dans laquelle lesdits doigts sont libérés desdits éléments de verrouillage et autorisent le soulèvement dudit couvercle,
le passage d'une position à la position suivante se faisant au moins par rotation relative du couvercle par rapport au réceptacle, dans un même sens.

Cette boîte est remarquable en ce que lesdits éléments de verrouillage portent au moins un moyen de blocage des doigts en "position intermédiaire", qui s'oppose au retour en "première position", tout en autorisant le passage à la position suivante.

Grâce à la présence de ce moyen de blocage, on ne peut normalement pas ramener les réceptacle et couvercle dans la position précédente. Ce "verrouillage" assure le manipulateur du "statut" de la boîte en "position intermédiaire".

Par ailleurs, selon d'autres caractéristiques avantageuses et non limitatives :
- ledit moyen de blocage est un cliquet anti-retour ;
- lesdits doigts présentent une région formant angle rentrant, apte à former butée pour le cliquet ;
- ledit cliquet anti-retour est escamotable ;
- ledit chemin de came est délimité par le muret lui même et par une "oreille" de matière solidaire du muret, ce muret et cette "oreille" délimitant transversalement, c'est-à-dire selon une direction généralement perpendiculaire à la direction de déplacement relative du doigt, un couloir pour l'entrée dudit doigt et son guidage le long du chemin de came entre les "première position" et "position finale" ;
- elle comporte des moyens indicateurs disposés à l'entrée dudit couloir, et/ou à sa sortie, dont l'état initial est modifié lors du passage de la "première position" à la "position intermédiaire", respectivement de la "position intermédiaire" à la "position finale", ce changement d'état étant perceptible visuellement ;
- lesdits moyens indicateurs comprennent au moins une languette solidaire du muret et de l'oreille, qui, dans l'état modifié, est détaché de l'un desdits muret et oreille ;
- dans l'état modifié, ladite languette est solidaire de ladite oreille et est immobilisée en position relevée ;
- ladite oreille est escamotable vers l'extérieur, c'est-à-dire dans une direction généralement opposée au couvercle ;
- ledit autre muret porte une excroissance qui, lors du passage de la "position intermédiaire" à la "position finale", exerce un effort sur ladite oreille et l'escamote.

D'autres caractéristiques et avantages de l'invention apparaitront à la lecture de la description détaillée qui va suivre de modes de réalisation préférés.

Cette description sera faite en référence aux dessins annexés dans lesquels :
- la figure 1 est une vue en perspective d'un premier mode de réalisation d'une boîte de Petri selon l'invention,
- la figure 3 est une vue en perspective du réceptacle de la boîte de Petri de la figure 1, sans son couvercle,
- la figure 5 est une vue en perspective dudit couvercle seul,

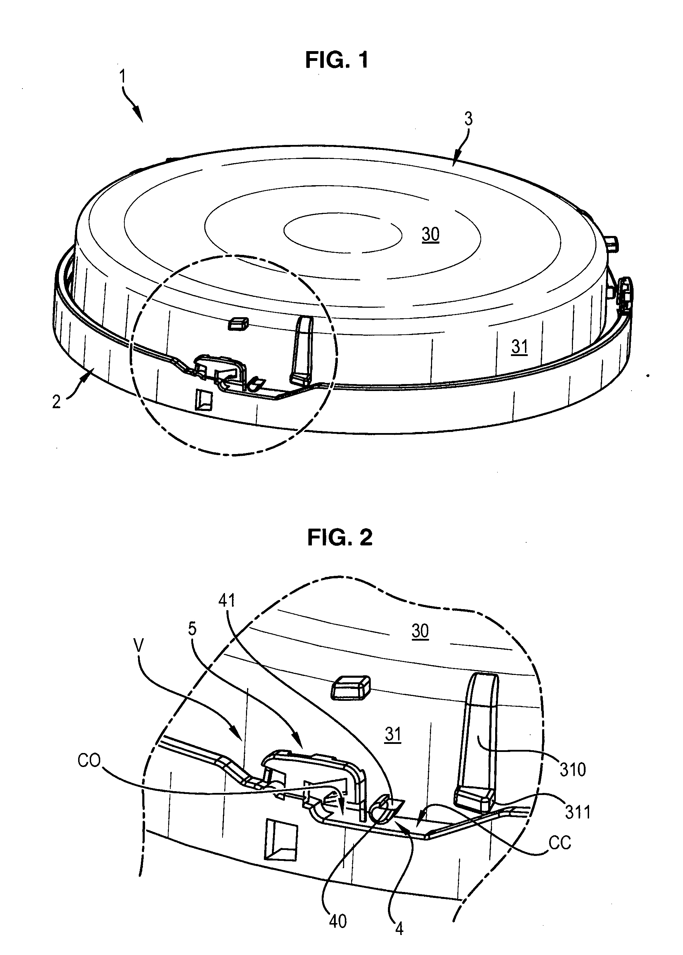

- les figures 2, 4 et 6 sont des vues agrandies des régions entourées d'un cercle, visible aux figures 1, 3 et 5,

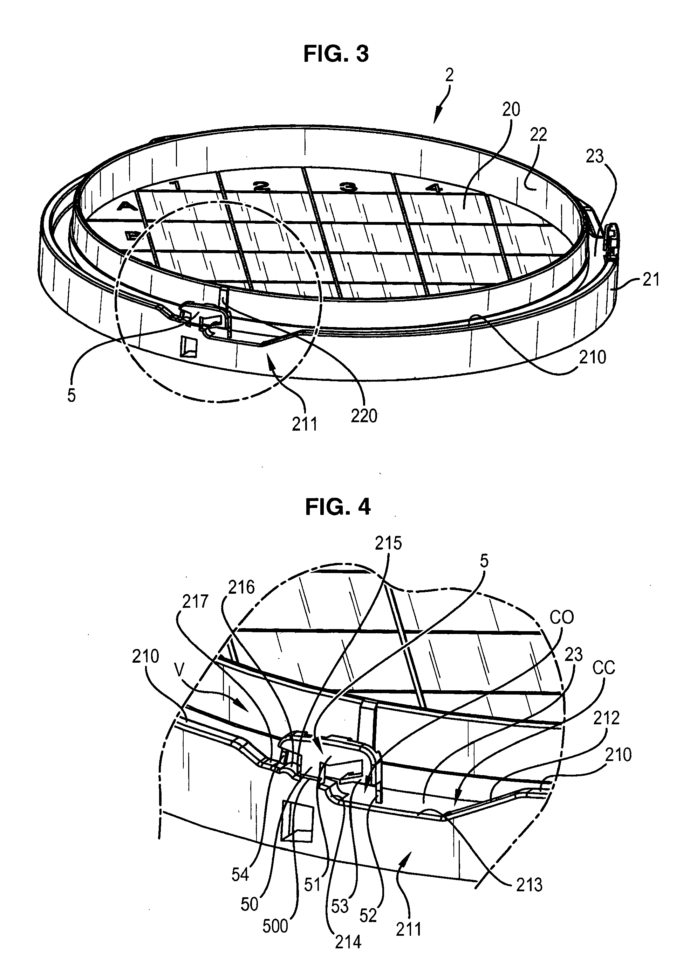

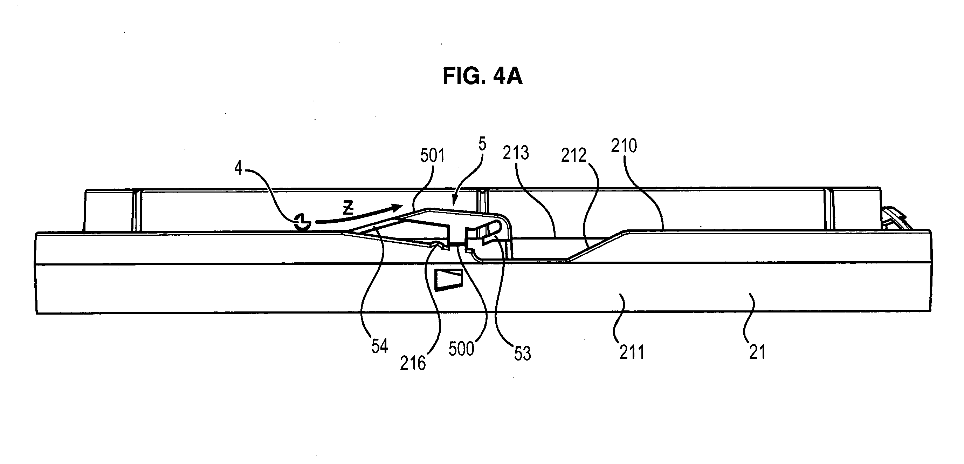

- la figure 4A est une vue de face d'un réceptacle pourvu d'une variante de réalisation de moyens de verrouillage,

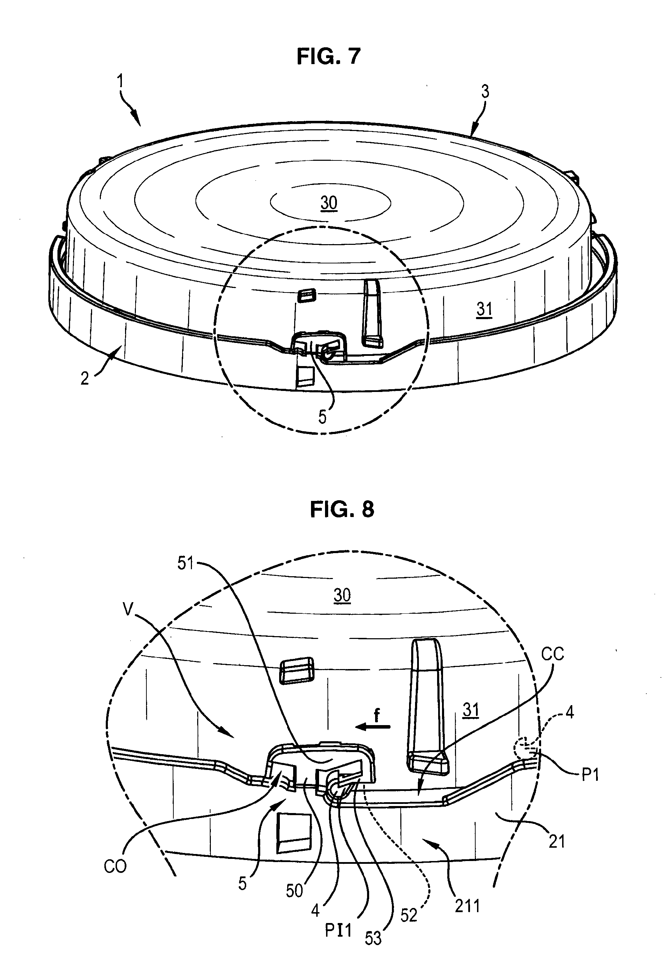

- la figure 7 est une vue analogue à la figure 1 montrant un positionnement particulier des couvercle et réceptacle l'un par rapport à l'autre, en l'occurrence dans une position dite "intermédiaire", verrouillée non ventilée,
- la figure 8 est un agrandissement de la zone repérée par un cercle à la figure 7,

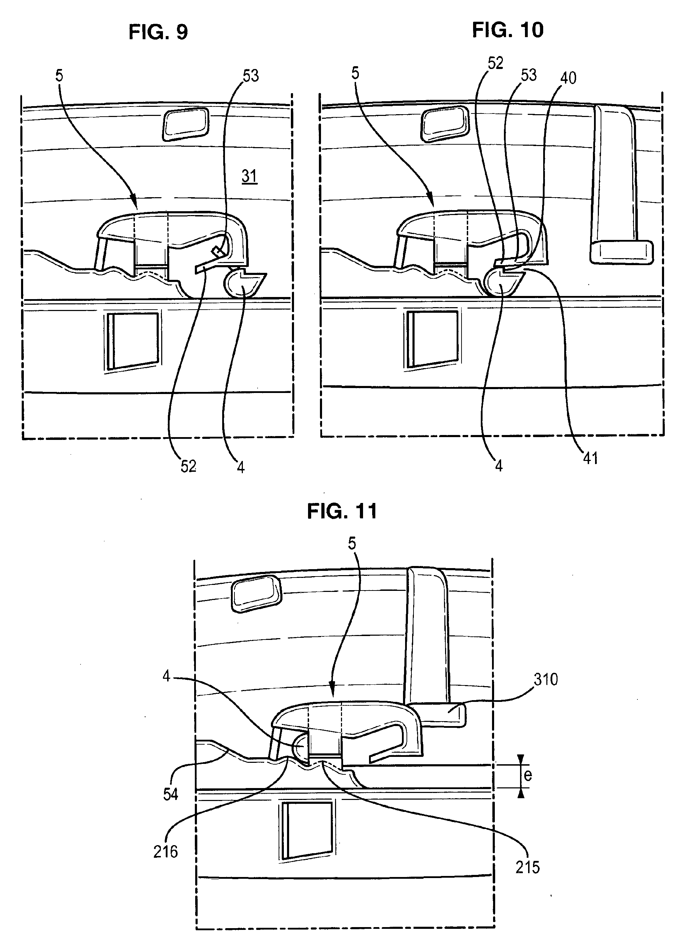

- les figures 9 à 11 sont des vues de détail du couvercle et du réceptacle, dans différentes positions relatives, dans lesquelles un doigt de verrouillage occupe des positions qui seront détaillées plus loin,

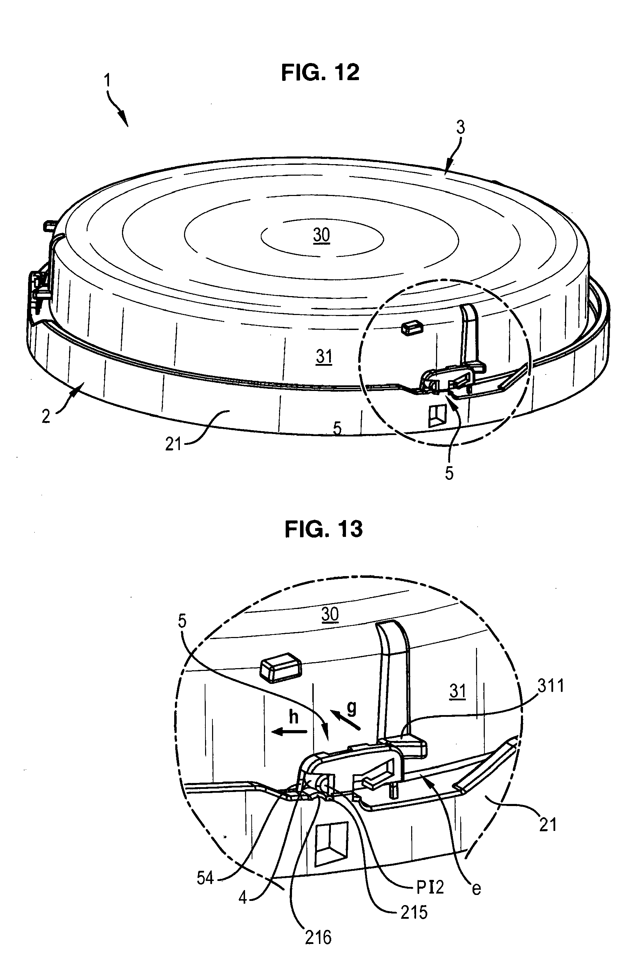

- les figures 12 et 13 sont des vues de la boîte de Petri dans la position illustrée partiellement à la figure 11,

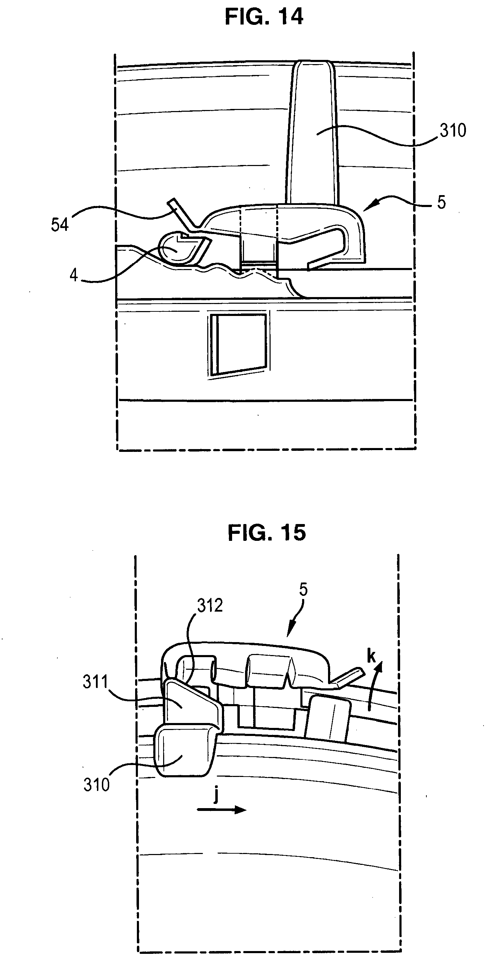

- la figure 14 est vue de détail du positionnement relatif du couvercle et du réceptacle, dans une position finale dite "de lecture",
- la figure 15 est une vue de dessus correspondant à la position de la figure 14,

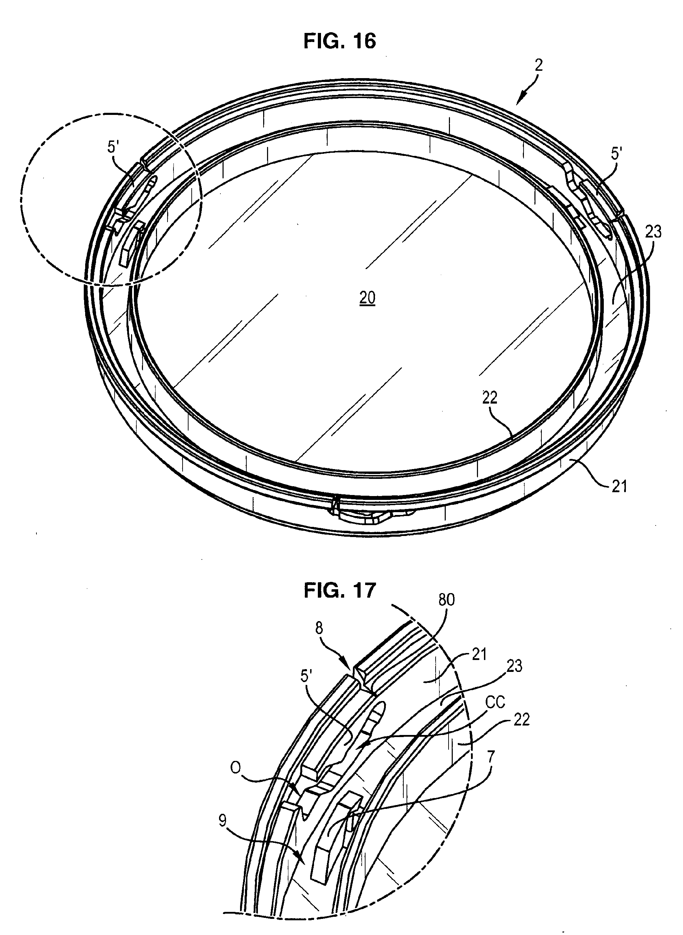

- la figure 16 est une vue en perspective d'un réceptacle conforme à un autre mode de réalisation de l'invention,
- la figure 17 est une vue agrandie de la zone repérée par un cercle à la vue précédente,

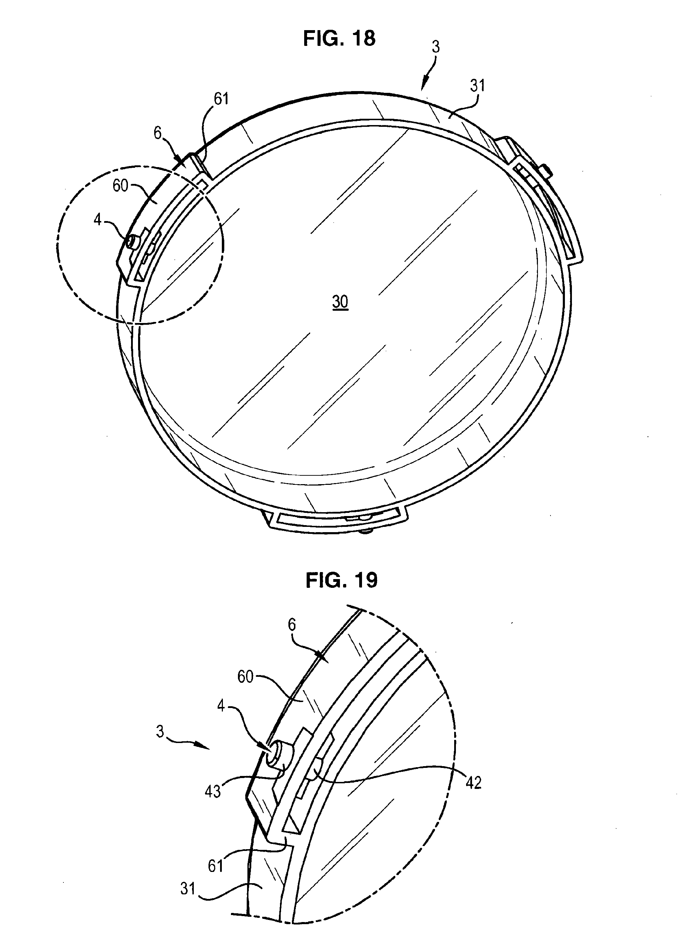

- la figure 18 est une vue en perspective, de dessous, du couvercle de ce deuxième mode de réalisation,
- la figure 19 est une vue agrandie de la zone repérée par un cercle sur la vue précédente,

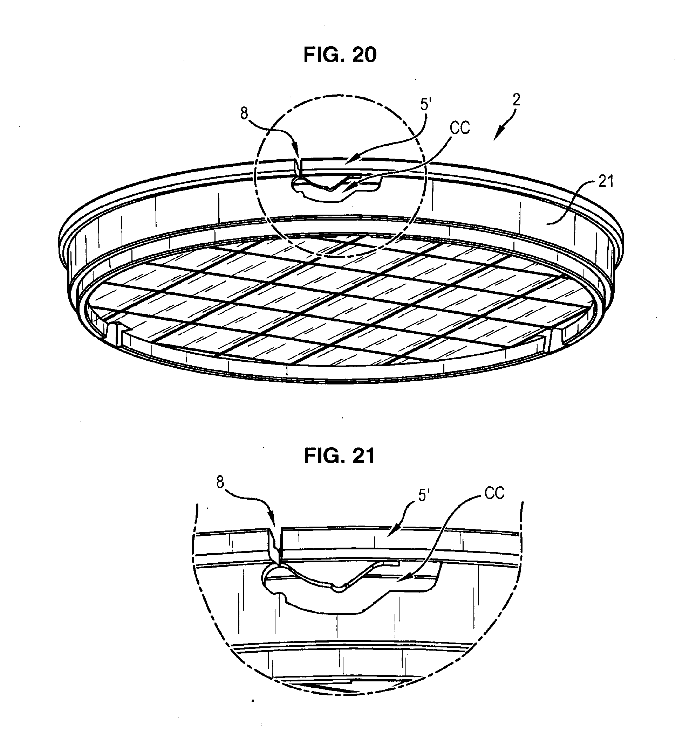

- la figure 20 est une vue en perspective du réceptacle de la figure 16, selon un autre angle de vue,
- la figure 21 est une vue agrandie de la zone repérée par un cercle à la figure précédente,

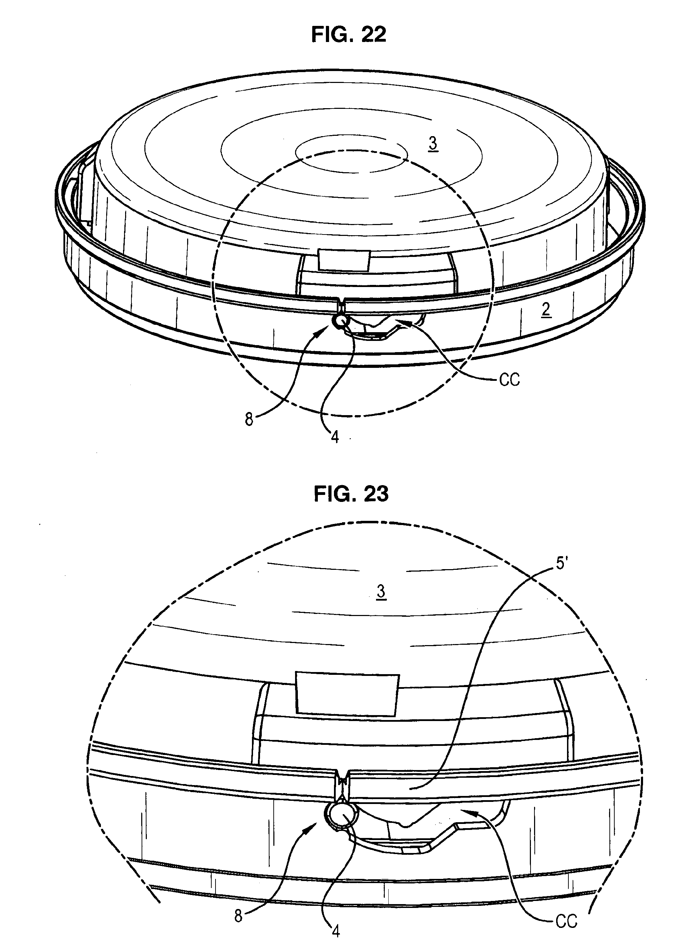

- la figure 22 est une vue en perspective des couvercle et réceptacle assemblés, dans une position particulière correspondant à une position verrouillée et ventilée,
- la figure 23 est une vue agrandie de la zone repérée par un cercle à la figure précédente.

On se reportera maintenant aux figures 1 à 15 pour détailler la structure du premier mode de réalisation de la boîte de Petri selon l'invention.

Comme cela est plus particulièrement visible aux figures 1, 3 et 5, la boîte de Petri 1 est constituée d'un réceptacle 2, et d'un couvercle 3, par exemple tous les deux en une matière plastique transparente telle que du polystyrène cristal.

Le réceptacle 2 présente une forme de révolution et comporte un fond 20 à contour circulaire, dont la surface supérieure est plane et dont la périphérie est délimitée verticalement par un premier muret 22, dit "muret intérieur".

Ainsi que cela est particulièrement visible à la figure 3, ce muret 22, présente, sur sa face externe, de petites surépaisseurs 220 qui présentent la forme de petits piliers et dont expliquera plus loin la fonction.

Le fond 20 se poursuit radialement au-delà du premier muret, pour constituer un chemin périphérique 23, lui-même délimité par ledit muret 22, ainsi qu'un second muret 21 dit "muret extérieur", de forme généralement cylindrique.

Ce muret extérieur 21 présente des dimensions telles que son sommet, matérialisé par la surface plane référencée 210, parallèle au fond 20, se situe à un niveau inférieur au sommet du premier muret 22.

En revanche, ce muret extérieur 21 se poursuit en deçà du fond 20. Autrement dit, il se poursuit vers le bas au-delà du fond 20 et du chemin périphérique 23.

Comme cela peut être vu notamment en examinant les figures 2 et 4, on constate que le muret extérieur 21 présente, dans des régions localisées, des échancrures 211 dans lesquelles sa hauteur nominale est largement diminuée.

En l'occurrence, dans le mode de réalisation représenté, ces échancrures sont au nombre de trois et sont angulairement équidistantes l'une de l'autre. Elles se trouvent placées en face des surépaisseurs formant piliers 220.

Ces échancrures 211, lorsqu'on considère les figures 2 et 4 sont formées, de la droite vers la gauche de ces figures, d'un tronçon rectiligne 212 à pente descendante, d'un tronçon horizontal et plan 213, qui est au même niveau que le chemin 23 précité.

Cette échancrure se poursuit par une zone courbe 214 à pente ascendante, qui se poursuit par deux régions successives convexes 216, qui encadrent un creux 215.

Enfin, deux zones 217 et 218 à pente ascendante se raccordent au sommet 210 du muret 21, au même niveau qu'initialement.

Tous ces tronçons et zones 212 à 218 forment un chemin de came CC.

Dans ces régions échancrées, et particulièrement et entre les zones 214 et 215 s'élèvent verticalement une "oreille" 5, venue de moulage avec le réceptacle 2, qui est raccordée au muret 21 par un pilier 50. Dans ce mode de réalisation, le pilier présente la particularité, de comporter une zone d'affaiblissement mécanique 500, par exemple sous la forme d'une rainure qui court transversalement à sa base.

A ce pilier se raccorde, selon une direction qui est généralement celle dudit muret, le corps 51 de l'oreille 5.

Le chemin de came CC et l'oreille 5 délimitent un couloir CO et constituent un élément de verrouillage V.

Ce corps 51, dans sa partie "amont", tournée vers la droite de la figure 4 se prolonge par une languette 52 qui est solidaire dudit corps 51, mais également du réceptacle 2.

Cette languette, telle que nous l'expliquerons plus loin, présente la particularité de pouvoir présenter un état modifié dans lequel elle est détachée, soit du muret 21, soit de l'oreille 5. De préférence, dans cet état modifié, la languette 52 est solidaire de l'oreille 5 et est immobilisée en position relevée.

Le corps 51 comporte par ailleurs un cliquet anti-retour 53 qui est orienté en biais vers le bas, en direction de la zone 214 précitée qui présente une pente ascendante courbe.

Là encore, nous reviendrons sur la structure et le fonctionnement de ce cliquet anti-retour, plus loin dans la description.

L'extrémité opposée dite "aval" du corps 51 comporte également une languette 54 qui présente les mêmes caractéristiques que celles référencées 52 et déjà décrites.

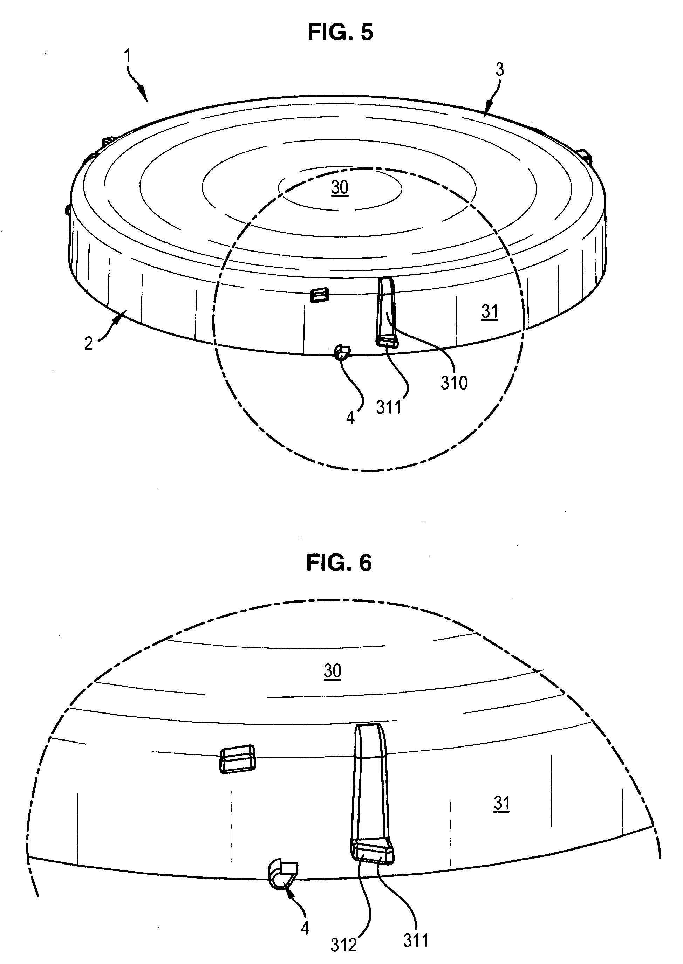

En se reportant maintenant notamment à la figure 5, on constate que le couvercle 3 porte, sur la face externe de son muret périphérique 31, des doigts équidistants référencés 4, et au nombre de trois dans le mode de réalisation illustré.

Ces doigts 4 font saillie radialement vers l'extérieur et s'étendent en position horizontale. Ils sont situés juste en bas du muret 31, le long de son rebord inférieur.

Ainsi que cela est plus particulièrement visible aux figures 2 et 4, ces doigts présentent une section transversale sensiblement ovoïde, avec un angle rentrant 40, dont on expliquera plus loin la fonction. Dans un mode de réalisation différent, la section transversale pourrait être circulaire.

Cet angle 40 rentrant se situe au niveau d'une découpe 41 prévue dans le quart supérieur droit du doigt 4.

Non loin de chaque doigt s'étend un pilier sensiblement vertical 310 qui fait corps avec le muret 31 et en constitue une surépaisseur.

A la base de cette surépaisseur s'étend radialement vers l'extérieur une excroissance 311 présentant un pan coupé référencé 312.

Comme le montrent plus particulièrement les figures 5 et 6, l'excroissance 311 se situe à un niveau légèrement supérieur à celui du doigt 4 voisin et est disposé à la droite de celui-ci quand on considère lesdites figures.

Les hauteurs respectives des murets 22 du réceptacle et 31 du couvercle sont telles que ce couvercle 3 peut reposer par le bord inférieur du muret 31, contre le fond 23 du réceptacle, entre les murets 21 et 22. C'est la position représentée à la figure 1.

Nous allons maintenant expliquer comment on utilise une telle boite de Petri.

En l'occurrence, nous décrirons successivement quatre positions que le réceptacle et le couvercle sont susceptibles d'occuper l'un par rapport à l'autre et reviendrons également sur la manière dont les équipements précédemment décrits coopèrent.

Avant même qu'une telle boîte de Petri ne soit utilisée et reçoive de la gélose ou un substrat nutritif, un utilisateur reçoit ladite boîte dans une position appelée première position et dite "préalable non verrouillée".

Dans cette position, le couvercle est positionné sur le réceptacle 2 de telle manière que les trois doigts 4 reposent sur le muret 21.

Cette position est repérée par une silhouette en traits mixtes à la figure 8 et est dénommée P1.

Dans une telle position, les doigts 4 sont "libres de mouvement" et le couvercle 3 est seulement retenu sur le réceptacle 2 par le fait que la face interne de son muret 31 opère une légère friction sur les piliers 220, dont est pourvu le muret interne 22.

Une fois après avoir procédé à l'enlèvement du couvercle, et aux opérations de prélèvement, on repositionne le couvercle 3 et on lui impartit une rotation sur une fraction de tour, de manière à ce que les doigts initialement en appui sur le sommet du muret 21 descende la pente 212 des échancrures 211 et viennent reposer sur la zone 213.

En continuant ce mouvement dans le même sens (flèche f, figure 8) on engage les doigts dans les moyens de verrouillage intégrés à l'oreille 5.

Plus précisément, et en référence aux figures 2, 9 et 10, on fait subir au couvercle ledit mouvement de rotation jusqu'à ce que chacun des doigts 4 soit positionné face à l'oreille 5 correspondante, juste devant la languette 52 (position de la figure 2).

En continuant le mouvement, cet indicateur que constitue la languette 52 est poussée et se détache du muret 31 pour se relever, comme le montre la figure 9.

Ce changement d'état de la languette 52 permet donc de rendre visuellement compte du fait que les couvercles 3 et réceptacle 2 ne sont plus dans la "première position" dite "préalable non verrouillée" mais dans une "position intermédiaire".

Conformément à l'invention, les éléments de verrouillage V de la boîte de Petri comportent un moyen de blocage des doigts 4 en position intermédiaire, qui s'opposent au retour en "première position", tout en autorisant le passage à la position suivante.

En l'occurrence, et en référence à la figure 10, lorsque le mouvement est poursuivi dans le même sens, le sommet du doigt 4 relève le cliquet anti-retour 53 jusqu'à ce que le doigt le franchisse et que ledit cliquet vienne se loger dans l'angle rentrant 40. On se trouve alors dans la "position intermédiaire" "verrouillée non ventilée", référence PI1 à la figure 8.

Dans cette position verrouillée, le muret 31 est en contact avec le fond du réceptacle 2 (chemin périphérique 23), de sorte que l'espace interne de la boîte de Petri est isolé du milieu extérieur.

Dans cette position intermédiaire, le cliquet anti-retour 53 s'oppose à un mouvement du doigt 4 dans une direction opposée à celle qui lui a été donnée initialement.

Ainsi, on a donc l'assurance, toujours de manière visible de l'extérieur, de la position verrouillée de la boîte de Petri.

Si par mégarde, un opérateur tentait de ramener le couvercle 3 et le réceptacle 2 dans la position préalable PI, il serait alors obligé d'agir à l'encontre du cliquet anti-retour, en escamotant celui-ci.

Par le terme "escamoté", on entend que le cliquet peut être amené dans une position dans laquelle il ne s'oppose plus au mouvement du doigt 4, en particulier par relèvement, grâce à une zone de faiblesse mécanique formant articulation.

Toutefois, si une telle manipulation a été effectuée, le positionnement relevé du cliquet renseigne alors immédiatement de la manipulation qui a été effectuée.

Dans le cas illustré ici et bien que cela ne soit pas obligé, il est possible de déplacer encore le doigt 4 pour lui faire occuper une autre position intermédiaire dite "verrouillée et ventilée" dans laquelle les doigts 4 sont toujours reçus dans les éléments de verrouillage V mais où le réceptacle 2 n'est plus en contact intime avec le couvercle 3, de sorte que l'espace interne qu'ils délimitent n'est plus isolé de milieu extérieur.

Cette position est illustrée aux figures 11 et 13 et est obtenue en déplaçant toujours le couvercle dans le même sens et en lui impartissant en plus un mouvement de translation vers le haut (flèches g et h).

Ce mouvement permet aux doigts de franchir la rampe 214 de manière à ce qu'ils viennent une position stable matérialisée par la zone 215 encadrée par les deux parties bombées 216.

Bien que cela ne soit pas représenté dans ce mode de réalisation, on pourrait très bien envisager que l'oreille 5 porte dans cette région un cliquet similaire au cliquet 53 déjà décrit, de manière à constituer un moyen de blocage des doigts dans cette position intermédiaire PI2, tout en s'opposant au retour en première position PI1.

Dans cette position intermédiaire PI2, le couvercle est soulevé d'une hauteur e correspondant à la différence de niveau entre la zone 215 et la zone 213, cet espace e étant mis à contribution pour ventiler l'intérieur de la boîte de Petri.

Lorsque la culture est finie et qu'un opérateur souhaite procéder à l'enlèvement du couvercle pour avoir directement accès à l'intérieur du réceptacle, il fait occuper au réceptacle 2 des couvercles 3 une dernière position finale dite "de lecture" dans laquelle les doigts 4 sont libérés des éléments de verrouillage V et autorisent le soulèvement du couvercle 3.

Ceci se fait à nouveau par un mouvement rotatif et relatif du couvercle 3 par rapport au fond 2, toujours dans le même sens. Ce faisant, chaque doigt 4 vient forcer contre la seconde languette 54 dont l'état initial est modifié et qui s'escamote tout en restant solidaire, en position relevée, de l'oreille 5. A nouveau, le passage d'une position à l'autre est visible et identifiable.

Durant cette opération et comme le montre plus particulièrement la comparaison des figures 11, 14 et 15, l'excroissance 311 portée par chacun des piliers 310 vient interférer avec l'oreille 5 correspondante, qui, en raison de la zone de faible résistance 500, se plie et s'escamote vers l'extérieur.

Ainsi, cette position escamotée est l'indication indéniable du passage de la précédente position intermédiaire à la position finale.

Si cette position était malencontreusement obtenue par une erreur de manipulation, alors l'escamotage de l'oreille 5 renseignerait immédiatement du statut de la boîte, ce qui permettrait automatiquement sa mise au rébus puisque non conforme aux exigences en la matière.

Comme indiqué plus haut, dans ce mode de réalisation, il est prévu deux positions intermédiaires dites "ventilée" et "non ventilée".

Toutefois, il est possible d'envisager des boîtes de Petri qui permettraient de n'obtenir qu'une seule de ces positions intermédiaires.

Le réceptacle de la figure 4A présente sensiblement la même structure que celle des figures précédemment décrites.

Toutefois, "l'oreille" 5 se différencie essentiellement de celle du mode de réalisation précédent par la forme de la face supérieure de cette dernière, qui présente une forme anguleuse, avec une partie terminale 501 qui est orientée en biais, en direction du rebord du muret 21.

Par ailleurs, la seconde languette 54 s'étend dans la continuité de la partie terminale 501.

De cette manière, on donne plus de résistance aux efforts à cette languette, de sorte qu'elle s'escamote uniquement si elle est volontairement sollicitée.

Par ailleurs, au moment où le couvercle de la boîte de Petri est positionné sur le réceptacle, il se peut que le doigt 4 soit positionné, non pas, en amont de l'oreille 5, mais en aval. Dans ce cas, en opérant une rotation dans le sens anti-horaire du couvercle par rapport au réceptacle, comme le montre la flèche Z, le doigt 4 est déplacé le long de la languette 4, qui fait fonction de tremplin.

Aux figures 16 à 23 est décrit un second mode de réalisation de la boîte de Petri.

Celui-ci se rapproche très fortement du mode de réalisation déjà décrit, notamment en ce qui concerne la structure du réceptacle 2.

En l'occurrence, la paroi de fond 20 se raccorde à deux murets cylindriques extérieur 21 et intérieur 22, séparés par un chemin périphérique annulaire 23.

En trois zones équidistantes angulairement, le muret extérieur 21 porte, sous forme de découpes réalisées dans l'épaisseur du muret, trois chemins de came CC accessibles par le sommet du muret, via une ouverture O.

Ainsi que cela est visible sur la figure 17 notamment le chemin de came CC est délimité vers le haut par une oreille 5' qui vient de matière avec le muret 21.

A l'opposé de l'ouverture O précitée, cette oreille 5' présente une découpe partielle 8 et ne tient au reste du muret que par une petite bande de matière référencée 80.

Comme le montre encore cette figure, ce réceptacle comporte un moyen de blocage en forme de cliquet anti-retour 7 qui n'est pas intégré au sein de l'oreille 5' comme dans le premier mode de réalisation, mais s'étend au niveau du chemin 23 séparant les deux murets précités.

Le cliquet est séparé du muret extérieur par un passage 9 à profil courbe, dont on expliquera la fonction.

Aux figures 18 et 19 est visible le couvercle 3 qui est destiné à venir coopérer avec le réceptacle qui vient d'être décrit.

Comme dans le mode de réalisation précédent, ce couvercle comporte une paroi de fond 30 et un muret périphérique 31.

En trois régions équidistantes angulairement, la paroi externe du muret 31 porte une cloison additionnelle 6 dont le corps 60 est situé à distance constante du muret 31 et qui se raccorde à ce dernier par deux pattes opposées 61.

La courbure et l'épaisseur du corps 60 correspondent sensiblement, au jeu près aux dimensions de l'espace formant couloir 9 qui a été décrit en référence à la figure 17.

Du muret 31 s'étendent, à travers la cloison additionnelle 6 des doigts 4, la partie située entre le muret proprement dit et la cloison 6 étant référencée 42, tandis que l'extrémité formant saillie extérieure est référencée 43.

Les dimensions des doigts 4 sont prévues pour que l'on puisse les engager, lorsqu'ils sont positionnés à cet effet, dans l'ouverture O du chemin de came précité.

Ce faisant, et dès lors que l'on aura déplacé le couvercle par rapport au réceptacle, le cliquet 7 va s'opposer au retour du réceptacle 2 et du couvercle 3 dans la position précédente à savoir ladite "première position".

Toutefois, si un effort important est réalisé dans ce sens, alors le cliquet est prévu pour s'escamoter, voire se casser. Grâce à cette caractéristique, on a alors un indicateur visible de la manipulation de la boîte.

Lorsque l'on a fait subir au couvercle, relativement au réceptacle, l'ensemble des positions désirées, on cherche alors à leur faire occuper la position finale déjà décrite dans laquelle les doigts 4 ne sont plus verrouillés et autorisent le soulèvement du couvercle 3.

Ceci est rendu possible par soulèvement du couvercle quand les doigts se trouvent à l'extrémité du chemin de came CC, juste en regard de la découpe précitée (découpe partielle 8).

En raison de la faible tenue mécanique de cette zone, il est possible de détacher au moins partiellement l'oreille 5' du muret 21, rendant possible la libération des doigts 4.

On a alors l'information visuelle selon laquelle la boîte de Petri a occupé à un moment donné, la position finale dite "de lecture".

Dans ces conditions, même si les doigts étaient réengagés sur les chemins de came CC, la modification d'état de l'oreille 5' serait visible et montrerait qu'une manipulation préalable a été mise en oeuvre.

## Revendications

1. Boite de Pétri (1), qui est constituée d'un réceptacle (2) et d'un couvercle complémentaire (3), qui ont tous les deux une forme de révolution et qui sont chacun délimités par une paroi de fond (20 ; 30) et au moins un muret périphérique (21 ; 22 ; 31),
- un muret (21 ; 22 ; 31) du réceptacle (2) ou, respectivement, du couvercle (3) portant au moins deux doigts (4) équidistants angulairement, généralement parallèles à la paroi de fond (20 ; 30), qui font saillie radialement en direction du muret (31 ; 21 ; 22) du couvercle (3) ou, respectivement du réceptacle (2),
- tandis que le muret (21 ; 22 ; 31) dépourvu de doigts (4) porte un même nombre d'éléments de verrouillage (V) pourvus d'un chemin de came (CC) apte à recevoir lesdits doigts (4),
- ce réceptacle (2) et ce couvercle (3) pouvant occuper sélectivement l'une ou l'autre des positions différentes successives indiquées ci-dessous :
a/ une "première position" (P1) dite "préalable non verrouillée", dans laquelle lesdits doigts (4) ne sont pas reçus dans lesdits éléments de verrouillage (V) ;
b/ au moins une "position intermédiaire" choisie parmi :
- une position "verrouillée non ventilée" (PI1), dans laquelle lesdits doigts (4) sont reçus dans lesdits éléments de verrouillage (V) et dans laquelle le réceptacle (2) est en contact intime avec le couvercle (3), de sorte que l'espace interne qu'ils délimitent est isolé du milieu extérieur, et
- une position "verrouillée et ventilée" (PI2) dans laquelle lesdits doigts (4) sont reçus dans lesdits éléments de verrouillage (V) et dans laquelle le réceptacle (2) n'est pas en contact intime avec le couvercle (3), de sorte que l'espace interne qu'ils délimitent n'est pas isolé du milieu extérieur ;
- c/ une "position finale" (PF) dite "de lecture", dans laquelle lesdits doigts (4) sont libérés desdits éléments de verrouillage (V) et autorisent le soulèvement dudit couvercle (3),
le passage d'une position à la position suivante se faisant au moins par rotation relative du couvercle (3) par rapport au réceptacle (2), dans un même sens,
**caractérisée par le fait que** lesdits éléments de verrouillage (V) portent au moins un moyen de blocage (53 ; 7) des doigts (4) en "position intermédiaire", qui s'oppose au retour en "première position", tout en autorisant le passage à la position suivante.

2. Boite selon la revendication 1, **caractérisée par le fait que** ledit moyen de blocage est un cliquet anti-retour (53 ; 7).

3. Boite selon la revendication 2, **caractérisée par le fait que** lesdits doigts (4) présentent une région formant angle rentrant (40), apte à former butée pour le cliquet (53).

4. Boite selon l'une des revendications 2 ou 3, **caractérisée par le fait que** ledit cliquet anti-retour (53) est escamotable.

5. Boite selon l'une des revendications précédentes, **caractérisée par le fait que** ledit chemin de came (CC) est délimité par le muret (21) lui même et par une "oreille" (5 ; 5') de matière solidaire du muret (21), ce muret et cette "oreille" délimitant transversalement, c'est-à-dire selon une direction généralement perpendiculaire à la direction de déplacement relative du doigt (4), un couloir (CO) pour l'entrée dudit doigt (4) et son guidage le long du chemin de came (CC) entre les "première position" et "position finale".

6. Boite selon la revendication 5, **caractérisée par le fait qu'**elle comporte des moyens indicateurs (52 ; 54) disposés à l'entrée dudit couloir (CO), et/ou à sa sortie, dont l'état initial est modifié lors du passage de la "première position" (PI1) à la "position intermédiaire" (PI1 ; PI2), respectivement de la "position intermédiaire" (PI1 ; PI2) à la "position finale" (PF), ce changement d'état étant perceptible visuellement.

7. Boite selon la revendication 6, **caractérisée par le fait que** lesdits moyens indicateurs comprennent au moins une languette (52 ; 54) solidaire du muret (21) et de l'oreille (5), qui, dans l'état modifié, est détaché de l'un desdits muret (21) et oreille (5).

8. Boite selon la revendication 7, **caractérisée par le fait que**, dans l'état modifié, ladite languette (52 ; 54) est solidaire de ladite oreille (5) et est immobilisée en position relevée.

9. Boite selon l'une des revendications 5 à 8, **caractérisée par le fait que** ladite oreille (5) est escamotable vers l'extérieur, c'est-à-dire dans une direction généralement opposée au couvercle (3).

10. Boite selon la revendication 9, **caractérisée par le fait que** ledit autre muret (31) porte une excroissance (311) qui, lors du passage de la "position intermédiaire" (PI1 ; PI2) à la "position finale" (PF), exerce un effort sur ladite oreille (5) et l'escamote.

## Patentansprüche

1. Petrischale (1), welche aus einem Boden (2) und einem komplementären Deckel (3) besteht, die beide eine Rotationsform auf-weisen und die jeweils durch eine Bodenwand (20; 30) und wenigstens eine Außenwand (21; 22; 31) begrenzt werden, wobei
- eine Wand (21; 22; 31) des Bodens (2) beziehungsweise des Deckels (3) wenigstens zwei Finger (4) in gleichem Winkelabstand allgemein parallel zu der Bodenwand (20; 30) aufweist, die radial in Richtung der Wand (31; 21; 22) des Deckels (3) beziehungsweise des Bodens (2) vorstehen,
- während die Wand (21; 22; 31), die keine Finger (4) besitzt, eine entsprechende Anzahl an Verriegelungselementen (V) aufweist, die mit einer Nockenbahn (CC) ausgestattet sind, die geeignet ist, die Finger (4) aufzunehmen,
- wobei der Boden (2) und der Deckel (3) selektiv die eine oder andere von aufeinander folgenden verschiedenen Positionen, die im Folgenden angegeben sind, annehmen können:
a/ eine "erste Position" (P1), bezeichnet als "vorangehend nicht verriegelt", in der die Finger (4) nicht von den Verriegelungselementen (V) aufgenommen werden;
b/ wenigstens eine "Zwischenposition", die ausgewählt ist aus:
- einer "nicht belüfteten verriegelten" Position (PI1), in der die Finger (4) von den Verriegelungselementen (V) aufgenommen werden und in der der Boden (2) in engem Kontakt mit dem Deckel (3) ist, der-art, dass der Innenraum, der von ihnen begrenzt wird, von der äußeren Umgebung isoliert ist, und
- einer "belüfteten verriegelten Position" (PI2), in der die Finger (4) von den Verriegelungselementen (V) aufgenommen werden und in der der Boden (2) nicht in engem Kontakt mit dem Deckel (3) ist, der-art, dass der Innenraum, der von ihnen begrenzt wird, nicht von der äußeren Umgebung isoliert ist;
c/ eine "Endposition" (PF), die als "Ablesungsposition" bezeichnet wird, in der die Finger (4) von den Verriegelungselementen (V) freigegeben sind und ein Anheben des Deckels (3) erlauben,
wobei der Übergang von einer Position in die nachfolgende Position wenigstens durch Relativdrehung des Deckels (3) in Bezug auf den Boden (2) in gleicher Richtung vonstatten geht,
**dadurch gekennzeichnet, dass** die Verriegelungselemente (V) wenigstens ein Arretierungsmittel (53; 7) zum Arretieren der Finger (4) in der "Zwischenposition" aufweisen, das einem Zurückkehren in die "erste Position" entgegensteht, jedoch einen Übergang in die nachfolgende Position ermöglicht.

2. Schale gemäß Anspruch 1, **dadurch gekennzeichnet, dass** das Arretierungsmittel eine Sperrklinke (53; 7) ist.

3. Schale gemäß Anspruch 2, **dadurch gekennzeichnet, dass** die Finger (4) einen Bereich aufweisen, der einen einspringenden Winkel (40) bildet, der geeignet ist, einen Anschlag für die Klinke (53) zu bilden.

4. Schale gemäß einem der Ansprüche 2 oder 3, **dadurch gekennzeichnet, dass** die Sperrklinke (53) einklappbar ist.

5. Schale gemäß einem der vorhergehenden Ansprüche, **dadurch gekennzeichnet, dass** die Nockenbahn (CC) durch die Wand (21) selbst und durch ein "Ohr" (5; 5'), das aus einem mit der Wand (21) fest verbundenen Stoff besteht, begrenzt wird, wobei diese Wand und dieses "Ohr" in Querrichtung, das heißt, in einer Richtung, die all-gemein senkrecht zu der Relativversetzungsrichtung des Fingers (4) verläuft, einen Gang (CO) für das Eintreten des Fingers (4) und seine Führung entlang der Nockenbahn (CC) zwischen der "ersten Position" und der "Endposition" begrenzen.

6. Schale gemäß Anspruch 5, **dadurch gekennzeichnet, dass** sie an dem Eingang des Ganges (CO) und/oder seinem Ausgang angeordnete Indikatormittel (52; 54) aufweist, deren Anfangszustand sich beim Übergang von der "ersten Position" (PI1) in die "Zwischenposition" (PI1; PI2) bzw. von der "Zwischenposition" (PI1; PI2) in die "Endposition" (PF) verändert, wobei diese Zustandsänderung visuell wahrnehmbar ist.

7. Schale gemäß Anspruch 6, **dadurch gekennzeichnet, dass** die Indikatormittel wenigstens eine Zunge (52; 54) umfassen, die mit der Wand (21) und dem Ohr (5) fest verbunden ist, und, im veränderten Zustand, von der Wand (21) oder dem Ohr (5) gelöst ist.

8. Schale gemäß Anspruch 7, **dadurch gekennzeichnet, dass** im veränderten Zustand die Zunge (52; 54) mit dem Ohr (5) verbunden ist und in angehobener Position blockiert ist.

9. Schale gemäß einem der Ansprüche 5 bis 8, **dadurch gekennzeichnet, dass** das Ohr (5) nach außen hin, das heißt, in eine Richtung, die dem Deckel (3) allgemein entgegensetzt ist, einziehbar ist.

10. Schale gemäß Anspruch 9, **dadurch gekennzeichnet, dass** die andere Wand (31) einen Vorsprung (311) aufweist, der bei einem Übergang von der "Zwischenposition" (PI1; PI2) in die "Endposition" (PF) eine Kraft auf das Ohr (5) ausübt und dieses versenkt.

## Claims

1. A Petri dish (1), which consists of a receptacle (2) and of an additional lid (3), which both have a revolution shape and which are each delimited by a bottom wall (20; 30) and at least one peripheral wall (21; 22; 31),
- a wall (21; 22; 31) of the receptacle (2) or respectively of the lid (3) bearing at least two angularly equidistant fingers (4) generally parallel to the bottom wall (20; 30), which radially protrude towards the wall (31; 21; 22) of the lid (3) or respectively of the receptacle (2),
- while the wall (21; 22; 31) without any fingers (4) bears a same number of locking members (V) provided with a cam path (CC) able to receive said fingers (4),
- this receptacle (2) and this lid (3) may selectively occupy either one of the successive different positions indicated below:
a/ a first position (P1), so-called "non-locked beforehand" position, in which said fingers (4) are not received in said locking members (V);
b/ at least one "intermediate position" selected from:
- a "non-ventilated locked" position (PI1), in which said fingers (4) are received in said locking members (V) and in which the receptacle (2) is in intimate contact with the lid (3), so that the internal space which they delimit is isolated from the outside medium, and
- a "locked and ventilated" position (PI2) in which said fingers (4) are received in said locking members (V) and in which the receptacle (2) is not in intimate contact with the lid (3), so that the internal space which they delimit is not isolated from the outside medium;
- c/ a "final position" (PF), so-called "read out" position, in which said fingers (4) are released from said locking members (V) and allow lifting of said lid (3),
the passing from one position to the next position being accomplished at least by a relative rotation of the lid (3) relatively to the receptacle (2), in a same direction,
**characterized by** the fact that said locking members (V) bear at least one means (53; 7) for blocking the fingers (4) in an "intermediate position", which opposes the return to the "first position", while allowing passing to the next position.

2. The dish according to claim 1, **characterized by** the fact that said blocking means is an anti-return ratchet (53; 7).

3. The dish according to claim 2, **characterized by** the fact that said fingers (4) have a region forming an interior angle (40), capable of forming an abutment for the ratchet (53).

4. The dish according to one of claims 2 or 3, **characterized by** the fact that said anti-return ratchet (53) is retractable.

5. The dish according to one of the preceding claims, **characterized by** the fact that said cam path (CC) is delimited by the wall (21) itself and by a "lug" (5; 5') of material secured to the wall (21), this wall and this "lug" delimiting transversely, i.e. along a direction generally perpendicular to the relative displacement direction of the finger (4), a corridor (CO) for entering said finger (4) and guiding it along the cam path (CC) between the "first position" and the "final position".

6. The dish according to claim 5, **characterized by** the fact that it includes indicating means (52; 54) positioned at the entry of said corridor (CO), and/or at its exit, the initial condition of which is modified upon passing from the "first position" (PI1) to the "intermediate position" (PI1; PI2), from the "intermediate position" (PI1; PI2) to the "final position" (PF), respectively, this change of state being visually perceptible.

7. The dish according to claim 6, **characterized by** the fact that said indicating means comprise at least one tab (52; 54) secured to the wall (21) and to the lug (5), which, in the modified state is detached from one of said wall (21) and lug (5).

8. The dish according to claim 7, **characterized by** the fact that in the modified state, said tab (52; 54) is secured to said lug (5) and is immobilized in a raised position.

9. The dish according to one of claims 5 to 8, **characterized by** the fact that said lug (5) is retractable outwards, i.e. in a direction generally opposite to the lid (3).

10. The dish according to claim 9, **characterized by** the fact that said other wall (31) bears a protrusion (311) which, during the passing from the "intermediate position" (PI1; PI2) to the "final position" (PF), exerts a force on said lug (5) and retracts it.
